(19) Europäisches Patentamt
European Patent Office
Office européen des brevets

(11) **EP 2 017 321 A1**

(12) **EUROPEAN PATENT APPLICATION**

(43) Date of publication:
**21.01.2009 Bulletin 2009/04**

(51) Int Cl.:
***C09K 8/582*** *(2006.01)* ***C12N 11/00*** *(2006.01)*

(21) Application number: **07112297.2**

(22) Date of filing: **11.07.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Stichting Deltares**
**2629 HD Delft (NL)**

(72) Inventors:
- **Latil-Collinet, Marie-Noëlle Lise**
  **2582 RB Den Haag (NL)**
- **van der Zon, Wilhelmus Hendrikus**
  **2521 ZL Den Haag (NL)**

(74) Representative: **van Loon, C.J.J. et al**
**Vereenigde**
**Johan de Wittlaan 7**
**2517 JR Den Haag (NL)**

(54) **A method for avoiding or reducing permeation of soil particles in a hydrocarbon well**

(57) The invention relates to a method for avoiding or reducing permeation of soil particles in a hydrocarbon reservoir, comprising applying (a) a urease producing micro-organism; (b) urea; and (c) optionally calcium or another ion for forming a carbonate mineral, to the hydrocarbon reservoir and allowing the micro-organism to degrade urea under formation of a carbonate mineral.

EP 2 017 321 A1

**Description**

The invention relates to a method for avoiding or reducing permeation of soil particles in a hydrocarbon well.

Sand permeation in hydrocarbon (oil, gas) reservoir formations that are not sufficiently consolidated is a problem that causes co-production of sand. This yields to an extra effort to remove that sand from the hydrocarbon stream and raises the production costs of the produced hydrocarbon. The production of sand in a hydrocarbon well may also, ultimately, result in a local collapse of the reservoir, due to poor consolidation of the sand. Further, fluids taken from a not sufficiently consolidated well usually comprise a lot of sand. As sand generally needs to be removed prior to further transport and/or processing, the removal adds to the production costs.

A conventional method to reduce permeation of soil particles (grains) in a subsurface formation is the use of a chemical such as a silicon polyhalide, e.g. as disclosed in US 4,506,732 or a epoxy-resin, e.g. as disclosed in US 4,168,257. Nowadays, use of such chemicals is discouraged for environmental reasons.

It is an object of the present invention to provide a novel method for reducing or avoiding permeation (migration) of soil particles, such as sand, in a hydrocarbon well which may serve as an alternative to known methods.

It is in particular an object to provide such method, whilst maintaining sufficient permeability of hydrocarbon in a geological formation from which the hydrocarbon is recovered.

It is in particular an object to provide such method which is suitable to consolidate soil particles, in particular sand particles, in a hydrocarbon well, such that hydrocarbon can be recovered from the well with a low concentration of soil particles, especially sand particles, or wherein such particles is even essentially absent, also at a high temperature, a high pressure, a high salinity of formation water, a low porosity of the geological formation, and/or at a high hydrocarbon concentration in the geological formation.

It is a further object to provide a method wherein the use of potentially hazardous non-natural chemicals can be avoided, or wherein after treatment soil particles are sufficiently consolidated in the hydrocarbon well in the absence of a non-natural consolidation agent, for instance a polymer or polymerisable compound such as a epoxy resin, or a siliocon polyhalide.

One or more objects which may be achieved in accordance with the invention will become apparent from the remainder of the description and/or the claims.

The inventors have realised that it is possible to meet one or more objects underlying the invention by forming a carbonate precipitate using a urease producing micro-organism in a hydrocarbon well in a specific way.

Accordingly, the present invention relates to a method for avoiding or reducing permeation of soil particles in a hydrocarbon reservoir, comprising applying effective amounts of (a) an urease producing micro-organism; (b) urea; and (c) optionally calcium or another ion for forming a carbonate mineral, to the reservoir and allowing the micro- organism to hydrolyse urea in the presence of a bi-valent metal ion under formation of a carbonate mineral.

Figure 1 shows a standard curve for the determination of ammonium concentration by the modified Nessler method.
Figure 2 is a graph showing the concentration of ammonium in the effluent and quantity of calcium carbonate precipitate in sand during the consolidation treatment, in an exemplary method of the invention.
Figure 3 is a graph showing the unconfined strength and porosity of the sand packed pre-wetted with production water and oil and consolidated with various number of batch treatment, in an exemplary method of the invention.

As used herein, the term hydrocarbon reservoir is used for a geological formation comprising a recoverable hydrocarbon, in particular oil, gas or a mixture thereof.

The term hydrocarbon well is used for a hydrocarbon reservoir in a geological formation, provided with means to recover the hydrocarbon from the reservoir. In particular such means includes a bore hole via which the hydrocarbon is recovered (a so called abstraction point). Usually, the reservoir treated in accordance with the invention is a hydrocarbon well, such as an oil well or gas well. The micro-organism, urea and/or cation for forming the mineral are usually applied via a bore hole, also used as an abstraction point during the recovery of the hydrocarbon.

The term "or" as used herein means "and/or" unless specified other wise.

The term "a" or "an" as used herein means "at least one" unless specified other wise.

When referring to a moiety (e.g. a compound, an ion, an additive etc.) in singular, the plural is meant to be included. Thus, when referring to a specific moiety, e.g. "ion", this means "at least one" of that moiety, e.g. "at least one ion", unless specified otherwise.

Advantageously a method of the invention can be employed without needing a non-natural chemical such as silicon polyhalide or epoxy or the like.

Further, in accordance with the invention the consolidation of soil is well controllable. For instance, the use of micro-organism allows consolidation (by mineralisation) to start, from a distance, i.e somewhat remote from the application point of the micro-organism, leaving the permeability at the application point(s) of the micro-organism - which can be the same as or near by the point(s) at which hydrocarbon is to be recovered (the bore hole(s)) - advantageously high for a good hydrocarbon recovery.

In particular, it is contemplated that the risk of clogging an abstraction point for the hydrocarbon is reduced in a method of the invention compared to using a non-natural chemical, in particular a polymer or polymerisable compound, such as an epoxy resin or silicon polyhalide, or even substantially avoided.

It is observed that the use of urease producing micro-organisms to treat a geological formation is known in the art. WO 2006/066326 relates to a method for forming a high strength cement utilising a microbial source of urease. The method comprises the step of combining in a permeable starting material effective amounts of (i) a urease producing micro-organism; (ii) urea; and (iii) calcium ions. The urease present in the organism hydrolyses urea into ammonium and carbon dioxide. The formation of ammonium will lead to an increase in pH, such that at least a considerable part of the carbon dioxide will will be present as carbonate. Carbonate precipitates with a bivalent metal ion, for instance calcium, to form a carbonate mineral, for instance calcite, if the concentrations of calcium and carbonate are sufficiently high.

It is stated that the method of WO 2006/066326 may be employed to produce cement for use in applications in a variety of applications, including in civil engineering, mining, environmental applications, restoration of heritage structures and the like, architectural applications and in the manufacture of special materials. In general terms, it is also mentioned that a method according to WO 2006/066326 may be used to increase the strength of a permeable starting material in the oil industry.

The present inventors have come to the conclusion that in particular for an improved consolidation of the reservoir around a bore hole of a hydrocarbon well such permeation of sand or other soil particles during recovery of hydrocarbon (oil or gas) from the well, the method conditions should be selected carefully.

On the one hand, soil particles (sand and/or other small soil-grains) should be sufficiently immobilised such that permeation is sufficiently reduced or avoided during normal use of the oil well. The skilled person will be able to determine what a sufficient immobilisition is, in a particular situation, based on common general knowledge the information disclosed herein and optionally some routine testing (e.g. making use of the Thick Wall Cylinder test).

On the other hand, the geological formation should retain sufficient permeability for the hydrocarbon in order to allow efficient hydrocarbon removal from the hydrocarbon well.

In principle, a method of the invention may be used for consolidation in relatively shallow reservoirs, for instance in reservoirs at an (average) depth of about 400-1500 m, or of about 1000-1500 m, below the surface. In particular, the inventors have realised that specific subterranean conditions may require special measures, in order to provide a favourable consolidation, especially in a deep reservoir (with a relatively high temperature and pressure), in particular in a hydrocarbon reservoir at an (average) depth of about 1500 m or more below the surface, more in particular at a depth of about 1500-4000 m below the surface. For instance, temperature may be high, exceeding, e.g., 40 °C.

In particular the temperature may be 45 °C or more. The temperature may be up to 60 °C, or more. In particular if the temperature in the reservoir is relatively high, in particular above 60 °C, the reservoir may be cooled, if desired, by applying a cooling medium (*e.g.* cold water (0-20 °C). However, a method of the invention may usually suitably be employed without having to inject cold water, prior to applying the micro-organism.

Further, depending on the depth, the micro-organism may be exposed to a high pressure of, e.g., 80 bar or more. Further, the formation water in oil reservoirs may have an extremely high salinity. In particular the water may be saturated or over-saturated with salt.

It is the inventors' finding that this may have an adverse affect on the micro-organism and/or may cause formation of calcite at an excessive rate. Moreover, the porosity of the geological formation wherein the hydrocarbon is present may be relatively low.

Moreover, the presence of hydrocarbons may have an effect on the activity of one or more enzymes inside the micro-organism and/or a property of the treated formation, such as strength, porosity and/or effectiveness of the consolidation of the soil particles.

A method according to the present invention is in particular suitable to be used for a hydrocarbon well in a geological formation wherein one or more of such conditions exist.

In particular, a method of the invention may be used for a hydrocarbon well in a geological formation which comprises formation water having a salt concentration of at least 200 g/l. or at least 250 g/l ($Na^+$, chloride, carbonates, $Ca^{2+}$, $Mg^{2+}$, sulphate, and other inorganic ions). The salt concentration may in particular be up to about 400 g/l, up to 350 g/l or up to 300 g/l.

In particular, the temperature (average between temperature at the highest point and lowest point in the region to be treated) may be more than 30 °C, in particular at least 35 °C, at least 40 °C or at least 45 °C. In particular the temperature may be up to 110 °C, more in particular up to 90 °C or up to 70 °C.

In particular, the pressure (average between pressure at the highest point and lowest point in the region to be treated may be more than 50 bar, at least 70 bar or at least 80 bar. Said pressure may in particular be up to 150 bar, more in particular up to 130 bar.

A method of the invention is particularly suitable for treating a geological formation of which the initial porosity (before applying the micro-organism) is less than 35 vol. %, in particular up to 33 vol. %, more in particular up to 32 vol.

% or up to 30 vol. %. The porosity as used herein is the value as can be determined by a method described in "Permeability determination through NMR detection of acoustically induced fluid oscillation. Magnetic Resonance Imaging, Volume 24, Issue 9, Pages 1187-1201 W. Looyestijn, *et al.*"

Usually the initial porosity is at least 15 vol. % Preferably, the initial porosity is at least 20 vol. %, or at least 24 vol. %.

The initial permeability of the geological formation for a 2 wt. % KCl solution in water before applying the micro-organism usually is at least 1 Darcy. Usually it is up to 5 Darcy, more in particular up to 4.0 Darcy or up to 3.5 Darcy.

It is contemplated that in accordance with a method of the invention permeability may in particular be decreased by at least 10 %, at least 15 % or at least 20 %. In particular, permeability may be decreased by up to 50 % up to 40 % or up to 30 %.

For retaining a sufficient hydrocarbon recovery capacity, the permeability, after formation of the mineral in accordance with the invention should usually be is at least 0.01 Darcy, in particular at least 0.1 Darcy For a favourable oil recovery capacity, the permeability after formation of the mineral preferably is at least 0.5 Darcy, more preferably at least 1.0 Darcy. Usually a permeability of up to 5 Darcy, up to 4 Darcy or up to 3 Darcy is retained, with the proviso that in general a reduction in permeability is realised in accordance with the invention.

By a method of the invention, the formation wherein the hydrocarbon well is situated may be reinforced. In particular, after treatment the geological formation wherein the well is present may have a strength, as determined by Dutch standard NEN-5117, of more than 5 MPa, more in particular of at least 10 MPa, or at least 15 MPa. A strength of up to 20 MPa or higher is considered feasible.

The inventors have in particular come to the conclusion that especially if the micro-organism is introduced into a hydrocarbon reservoir before at least one component selected from urea and calcium, the risk of formation of calcite (or another mineral) at an excessive rate is reduced or even essentially absent, whilst sand permeation is sufficiently controlled and still allowing satisfactory oil recovery from the well.

Preferably, the micro-organisms are immobilised after they have been introduced. In particular, immobilisation may be accomplished by a method wherein according to the invention comprising

- forming a first zone comprising the micro-organism in the geological formation wherein the hydrocarbon well is present and allowing the first zone to move through at least part of the material;
- forming a second zone comprising an effective amount of a flocculating agent to the material and allowing the second zone to move through at least part of the material, wherein the zones are allowed to move such that the zones become at least partially overlapping and at least part of the bacteria flocculate, thereby becoming immobilised. In an embodiment, the second zone is formed directly after the first zone and both zones are allowed to move in the same direction through at least part of the material.

The first and the second zone may be provided by first pulse-injecting a liquid comprising the micro-organism and thereafter pulse-injecting a liquid comprising the flocculation agent.

Such a method of the invention is advantageous in the micro-organisms can be distributed and immobilised homogeneously in the part of the reservoir that is treated.

The flocculating agent may in particular be selected from the group of divalent cations, preferably from the group consisting of nickel-, zinc-, magnesium- and calcium ions. In a preferred embodiment, the flocculating agent also acts as a cation which forms the carbonate mineral with carbonate.

In a preferred embodiment the first and/or the second zone are moved through the material at a rate defined by a hydraulic gradient in the range of 0.1 to 2.0.

In a preferred embodiment, the urea is added after immobilisation of the micro organism, preferably with (additional) cations to form the mineral.

A suitable method for immobilisation of micro-organisms is described in more detail in the international application with application number PCT/NL/2006/000598, of which the contents are incorporated herein by reference.

The inventors further contemplate that it is advantageous to selective treat a relatively small region around a bore hole in the well. In particular, in an embodiment of the invention, at least the micro-organism is selectively applied to a region at least partially surrounding a bore hole in the oil well. Such selective application of the micro-organism may in particular be realised using an immobilisation method as described above.

In particular, the method of the invention may be employed to consolidate an area surrounding a bore hole, starting at distance of at least 0.2 m or at least 0.3 m from a bore hole. The distance may in particular be up to 2 m, up to 1.5 m or up to 1 m. Thus, consolidation may be effected in a ring-like manner around the bore hole.

In order to accomplish consolidation in a defined area around a bore hole, preferably, the microorganisms are introduced in the hydrocarbon well by injecting a suspension comprising a microorganism, followed by a liquid comprising the flocculating agent which spread the cells in the material (and whereby the suspension is diluted). In particular the volume to volume ratio of the suspension comprising the micro-organism to the liquid comprising the flocculation agent may be 10:90 to 50:50, preferably 20:80 to 50:50. Such method is particular advantageous for a good distribution of the bacteria in the material to be treated and, also to cause immobilization of the micro-organism.

The inventors have further realised that for favourably avoiding or reducing sand permeation, whilst maintaining good porosity of the geological formation wherein the well is present to allow satisfactory hydrocarbon recovery, it is preferred that the micro-organism, as immobilised, is present in the material in a relatively low concentration. In a method of the invention, applying the micro organisms in a relative low concentration will be sufficient to adequately immobilise loose grains in the formation.

A preferred concentration is based on the specific urease activity of the micro-organism under conditions existing at the average depth of the region to be treated in accordance with the invention (i.e. the average of the highest point and the lowest point in the region to be treated). Suitable concentrations may be based on laboratory conditions emulating these conditions and/or by extrapolation from standard laboratory conditions.

Usually, the micro-organism is provided at a concentration corresponding to a urea hydrolysis rate of up to 100 mM urea hydrolysed per minute (after applying the urea), at the temperature, pressure and salt concentration existing at the application point in the well.

Preferably, the urea hydrolysis rate under said conditions is up to 50 mM urea hydrolysed per minute, in particular up to 30 or up to 25 mM urea hydrolysed per minute (average). Usually the micro-organism is applied to provide an (average) hydrolysis rate or at least 5 mM urea hydrolysed per minute, based on the temperature, pressure and salt concentration existing at the application point in the well.

Alternatively a suitable rate may be chosen based on the following lab conditions: Preferably, a hydrolysis rate under laboratory conditions (i.e. 18 °C, normal pressure) is at least 0.2 mM urea hydrolysed per minute, (average) at least 2 mM urea hydrolysed per minute or at least 10 mM urea hydrolysed per minute (maximum), wherein the hydrolysis rate is preferably maintained at or above any of said preferred lower limits for at least 1 hour, more preferably for at least 10 hours, depending on the required strength improvement.

As a urease producing micro-organism, a urease producing bacterium is particular suitable. The bacterium may in particular be selected from the group consisting of *Sporosarcina,* more in particular *Sporosarcina pasteurii*.

Preferably the micro-organism is a mesophile, such as a *Sporosarcine Pasteurii,* or an extremophile, in particular an thermophile having its maximum urea hydrolysis rate (1.5 M urea in demineralised water, measured for 5 minutes) at a temperature of 40 °C or more, in particular of 45 °C or more, more in particular of 50 °C or more.

Such a micro-organism is in particular useful in a method of the invention, as the (average) temperature in the well is usually more than 30 ° C, in particular at least 35 °C, at least 40 °C or at least 45 °C, *e.g.* up to 110°C, in particular up to 90 °C, up to 80 °C or up to 80 °C. It should be noted that a preferred micro-organism, in particular a mesophile or thermophile used in a method of the invention is typically employed at a concentration to provide a urea hydrolysis rate at 25 °C of considerably less than 50 mM urea hydrolysed per minute (using 1.5 M urea as substrate). In particular, the hydrolysis rate at 25 °C (1 bar), usually is less than 10 mM urea hydrolysed per minute, in particular less than 5 mM urea hydrolysed per minute or less than 3 mM urea hydrolysed per minute.

As the micro-organism is usually employed at a concentration providing a relatively low hydrolysis rate, the micro-organism is usually applied in a dilute form.

The micro-organism may in particular be applied as a dispersion in an aqueous liquid. The term aqueous liquids is used herein for liquids wherein water is the predominant solvent, preferably essentially the only solvent. In particular it is preferred that 90-100 wt.% of non-solids in the liquid, more in particular 99-100wt.% of non-solids in the liquid is water.

The inventors have found that diluting a micro-organism culture in tap water or demineralised water may cause a fast release of urease from the micro-organism to the liquid phase. In general, this does not need to be a problem as extracellular urease substantially retains activity under conditions that are not as extreme as the conditions which may exist in a hydrocarbon well (such as high temperature, high salinity, high pressure). For instance, for extracellular urease from *Sporosarcina pasteurii* good activity is maintained at 20 °C. However, the inventors have acknowledged that under extreme conditions, such as at an elevated temperature, extracellular urease in tap water or demi-water looses activity relatively fast (within a few minutes at temperature above 45 °C). The inventors also found that it is possible to maintain urease activity for a prolonged time or at least reduce the decrease rate of the urease activity by diluting the micro-organism in saline solution.

Thus, in a preferred method of the invention the micro-organism is applied as a dispersion of the micro-organism in an aqueous liquid having an ionic strength of at least 0.05 M, in particular of at least 0.1 M, *e.g.* about 0.15 M. The concentration is usually up to 0.5 M, in particular up to 0.3 M or up to 0.2 M, although it is thought that a higher concentration may be employed, especially at high salinity of the formation water. As a salt for providing said ionic strength, in principle any salt can be used wherein the micro-organism remains sufficiently active. NaCl is particularly suitable.

The osmolarity of the aqueous liquid, preferably is at least 150 mOsm/l, at least 200 mOsm/l or at least 250 mOsm/l. The osmolarity preferably is up to 500 mOsm/l, up to 400 mOsm/l or up to 350 mOsm/l. Urea is preferably applied in a concentration of up to 1000 mM, in particular up to 500 mM, more in particular up to 200 mM. Preferably, the concentration is at least 50 mM or at least 100 mM.

Calcium or another cation for forming the mineral is optionally added. In particular in case the formation water has a high salinity, more in particular if it contains sufficient calcium ions or other ions to form a carbonate mineral with the carbonate formed by hydrolysis of urea such addition may be omitted. Calcium or the other cation may in principle by applied as any salt, as long as it is not unacceptably detrimental to the hydrolysis of the urea. In particular, a chloride or nitrate salt may be used. In an embodiment, production water is used as a cation source for the mineral. Production water is water from the geological formation (formation water) that is produced together with the hydrocarbon.

If added, the cation for forming the mineral is usually added in a concentration of up to 1000 mM , in particular up to 500 mM, more in particular up to 200 mM.

If present, the concentration of the cation for forming the mineral is usually at least 1 mM, in particular at least 2 mM or at least 5 mM. In particular in conditions, wherein the solubility of the formed carbonate is relatively high, in case the calcium concentration in the formation water is relatively high, and/or in case it is desired to also considerably strengthen the geological formation (near a bore hole), the concentration of calcium or another cation for forming the mineral may be at least 10 mM, at least 25 mM, at least 50 mM or at least 100 mM.

If added, calcium or the other cation is usually applied to provide a concentration which is about equi-molar, to the applied urea concentration. In particular the cation to form the mineral may be applied to provide concentration may be 0.5 to 2 times the urea concentration.

Urea (and optionally calcium or another cation) is usually added such that less than 60 g of carbonate mineral per litre of liquid comprising urea added to the well is formed. Preferably, urea (and optionally calcium or another cation) is added such that less than 30 g of carbonate mineral per litre of liquid comprising urea added to the well is formed, for an efficient hydrocarbon recovery In particular, urea (and optionally calcium or another cation) may be added such that up to 21 g of carbonate mineral per litre of liquid comprising urea added to the oil well is formed. Preferably at least ... g or at least ... of carbonate mineral per litre of liquid comprising urea (and optionally calcium or another cation) added to the oil well is formed, for a good consolidation of soil particles.

Further, the flow rate at which urea and/or calcium or other cation may be chosen within a specific range to achieve a specific degree of consolidation of sand or other soil particles. Preferably, urea and/or calcium or other cation are applied at a radial flow rate in the well of at least 2.5 cm/sec, in particular at least 7.5 cm/sec. The radial flow rate is preferably up to 50 cm/sec, or up to 25 cm/sec.

The invention is now illustrated by the following examples.

**Examples**

General Material and Methods

• Urease activity measurement

Urease is a specific enzyme which hydrolyzes urea (non-ionic species) to ammonium and carbonate (ionic species).

$$CO(NH_2)_2 + H_2O \rightarrow 2NH_4^+ + CO_3^{2-}$$

The resulting change in conductivity is used to monitor the rate of reaction.

5 ml of 6M urea is mixed to 13 ml water (final concentration 1.5 M). 2 ml of sample is added and the conductivity is measured at 1 minute intervals for 5 minutes.

The linear change in conductivity is plotted versus time.

$$Urease\ activity\ (mS/\min)\ =\ Slope\ \frac{\delta\,mS}{\delta\,t} \times dilution$$

• Ammonium concentration measurement

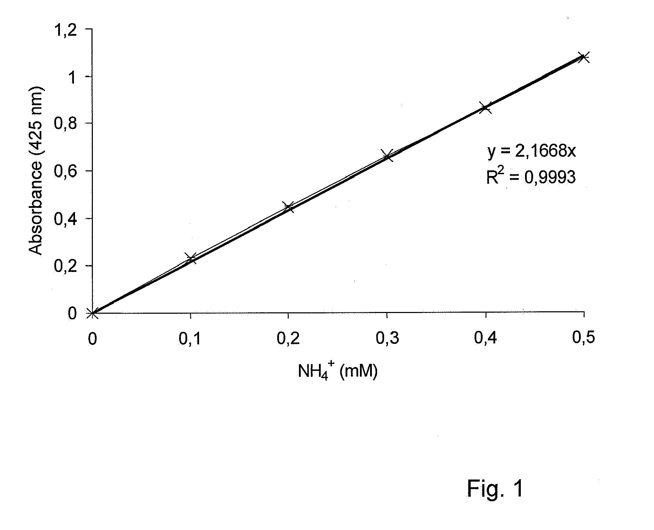

Ammonium concentration is measured with a modified Nessler metod. The sample is diluted in demineralized water in order to get an concentration in the range of 0-0.5 mmol/l. 100 $\mu$l of Nessler reagent is mixed with 2 ml of the diluted sample. After exactly 1 minute the absorbance is measured at 425nm. According to the standard curve, [NH4+] mmol/l = $Abs_{425nm}/2,17$ (Figure 1)

• Strength measurement

After treatment, the columns are cut free from the PVC tube with a low velocity horizontal saw and tested for unconfined compressive strength (conforming to Dutch standard NEN-5117).

**Example 1: continuous treatment**

Two packed-sand columns pre-wetted with production water and Oil have been successfully consolidated at 50°C and 80 bar.

Material and method

*Preparation of the columns:*

The experiment has been carried out in PVC columns with an inner diameter of 6.5 cm and a length of 18 cm. One centimeter at the top and at the bottom of the columns have been filled with a standard filter sand of 1 mm grain size. Between these two layers, a subrounded sand with a grain size distribution of 125-250 $\mu$m has been packed under saturation by stamping to get a maximum bulk density.

In order to simulate an oil reservoir, the columns have been flushed with two pores volume of production water followed by two pores volume of crude oil. The standard procedure before treating an oil well is to roughly clean up the sand formation with a solution of KCl and then diesel. Therefore the columns have been flushed with two pores volume of KCl 2% followed by two pores volume of diesel and finally two pores volume of KCl 2% again.

*Consolidation treatment:*

The columns have been flushed with one pore volume of a bacteria suspension of *Sporosarcina pasteurii* diluted in a solution of NaCl 0.9 g/l in order to adjust the urease activity of the solution at 2 mS/cm/min (see protocol of urease activity measurement). Immediately after, the columns have been flushed with one pore volume of a solution of 50 mmol/l of calcium chloride followed by a solution of urea and calcium chloride which contained 1 mol/l of each product. This solution has been injected through the columns for about five hours.
After the treatment the columns were flushed with demineralized water to remove the residual urea, calcium chloride and ammonium from the sand.

*Measurements:*

During the treatment, ammonium concentration in the effluent was monitored. the calcium carbonate contained was calculated by the following formula:

$$[\mathrm{CaCO3}]_{mg/cm3} = \tfrac{1}{2}[\mathrm{NH4}].\,Q.dt.MW_{\mathrm{CaCO3}}.V.10^3$$

Q = Flow rate of the Ca/urea solution [1/h]
dt = Ca/urea Flush time [h]
$MW_{\mathrm{CaCO3}}$ = 100 g/mol
V = volume of the sand packed [cm$^3$]

The permeability, the porosity and the UCS strength was measured after treatment.

*Results:*

As shown in the table below, the unconsolidated packed-sand columns, pre-wetted with production water and oil to simulate the an oil reservoir, have been successfully cemented after a continuous treatment of approximately 5 hours without dramatic loss of permeability.

| | Period of treatment | Porosity | UCS | CaCO₃ contained |
|---|---|---|---|---|
| | h | % | bar | mg/cm3 |
| column 1 | 5,31 | 19,3 | 80 | 313 |
| column 2 | 4,85 | 20,5 | 55 | 265 |

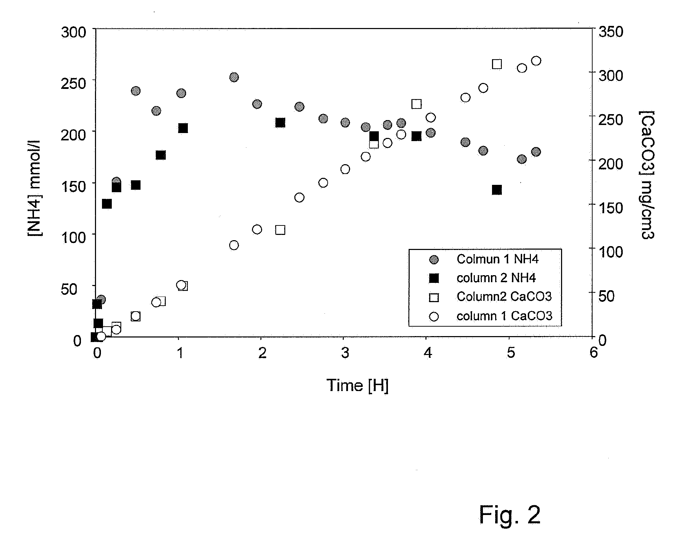

The ammonium concentration measured in the effluent during the treatment and the calculated quantity of calcium carbonate precipitated in the sand column is shown in Figure 2. Despite the extreme temperature, pressure and the presence of residual oil and production water, the urease remains active after more than five hours.

**Example 2: batch treatment**

Four packed-sand columns pre-wetted with production water and Oil have been consolidated at 50°C and 80 bar batchwise.

Material and method

*Preparation of the columns*

The four columns have been prepared with the same protocol as the one described in example 1.

*Consolidation treatment*

To immobilize the enzyme in the sand formation the columns have been flushed with one pore volume of a bacteria suspension of *Sporosarcina pasteurii* diluted in a solution of NaCl 0.9 g/l in order to adjust the urease activity of the solution at 2 mS/cm/min. Immediately after, the columns have been flushed with one pore volume of a solution of 50 mmol/l of calcium chloride following by one pore volume of a solution of urea and calcium chloride which contained 1 mol/l of each product. The columns have been kept half an hour at 50°C under 80 bar to let the reaction take place. Ammonium concentration has been measured in order to check if all the urea had been hydrolyzed. This batch treatment (injection of one pore volume of urea and calcium chloride solution) has been repeated several times under the same protocol. (column #1 : 3 batch treatment, column #2 : 4 batch treatments, column #3 : 5 batch treatments, column #4 : 8 batch treatments)

After the treatment the columns were flushed with demineralized water to remove the residual urea, calcium chloride and ammonium from the sand.

*Measurements*

Ammonium concentration has been measured at the end of each batch treatment. The permeability, the porosity and the UCS strength have been measured at the end of the whole treatment.

*Results*

| | Number of batch treatment | Ammonium concentration mmol/l | UCS bar | permeability darcy | porosity % |
|---|---|---|---|---|---|
| Column 1 | 3 | 2000 | 4 | | 0,35 |
| Column 2 | 5 | 2000 | 14,9 | | 0,306 |
| Column 3 | 7 | 2000 | 32,2 | | 0,264 |
| Column 4 | 9 | 2000 | 56,2 | | 0,223 |

| Column number | Number of batch treatment | Ammonium concentration (mmol/l) | UCS (bar) | Porosity (%) |
|---|---|---|---|---|
| 1 | 3 | 2000 | 4.0 | 35.0 |

(continued)

| Column number | Number of batch treatment | Ammonium concentration (mmol/l) | UCS (bar) | Porosity (%) |
|---|---|---|---|---|
| 2 | 5 | 2000 | 14.9 | 30.6 |
| 3 | 7 | 2000 | 32.2 | 26.4 |
| 4 | 9 | 2000 | 56.2 | 22.3 |

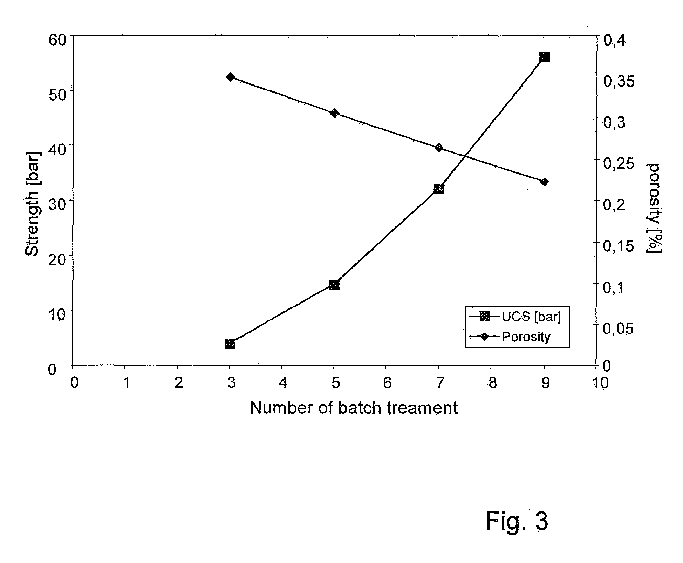

As shown in the Figure 3, the batch treatment method is a good option to avoid sand permeation in a oil reservoir during oil production. The number of batch treatment can be adjusted according the amount of consolidation needed (UCS).

**Claims**

1. A method for avoiding or reducing permeation of soil particles in a hydrocarbon reservoir, comprising applying (a) a urease producing micro-organism; (b) urea; and (c) optionally calcium or another ion for forming a carbonate mineral, to the hydrocarbon reservoir and allowing the micro-organism to degrade urea under formation of a carbonate mineral.

2. A method according to claim 1, wherein urea, and - if applied - the calcium or other ion for forming the mineral are applied after the micro-organism is applied.

3. A method according to claim 2, wherein the micro-organism is immobilised before applying the urea.

4. A method according to any of the preceding claims wherein the micro-organism is suspended in an aqueous liquid having an ionic strength of at least 0.05 M, in particular of 0.1-0.5 M.

5. A method according to any of the preceding claims wherein at least the micro-organism is applied in a region at least partially surrounding a bore hole in the hydrocarbon reservoir.

6. A method according to any of the preceding claims, wherein the micro-organism, the urea and - if applied the calcium or other ion for forming the mineral are applied via a bore hole in the reservoir.

7. A method according to any of the preceding claims, wherein the micro-organism is a mesophile or an extremophile, in particular a mesophile or thermophile having its maximum urea hydrolysis rate at a temperature exceeding 40 °C, more in particular of 50 °C or more.

8. A method according to any of the preceding claims, wherein the micro-organism is applied in a concentration to provide a urea hydrolysis rate in the range of 5 to 100 mM urea per minute, at the temperature, pressure, urea concentration and salt concentration existing at the application point in the oil well.

9. A method according to any of the preceding claims, wherein urea is applied in a concentration of up to 1000 mM , in particular of 50 to 500 mM, more in particular 100 to 200 mM.

10. A method according to any of the preceding claims, wherein less than 60 g of carbonate mineral per litre of liquid comprising urea added to the reservoir is formed.

11. A method according to any of the preceding claims, wherein calcium or the other cation is applied in a concentration of up to 1000 mM , in particular of 50 to 500 mM, more in particular 100 to 200 mM.

12. A method according to any of the preceding claims, wherein the reservoir comprises formation water having a salt concentration of at least 200 g/l.

13. A method according to any of the preceding claims, wherein the (average) temperature in the reservoir is more than 30 °C, in particular in the range of 35-110 °C, more in particular in the range of 40-70 °C.

14. A method according to any of the preceding claims, wherein the (average) pressure in the reservoir is more than 50 bar, in particular in the range of 70-150 bar, more in particular in the range of 80-130 bar.

15. A method according to any of the preceding claims, wherein the porosity of the geological formation wherein the reservoir is present is less than 35 vol. %, in particular in the range of 20-33 vol. %, more in particular in the range of 24-32 vol. %.

16. A method according to any of the preceding claims, wherein the permeability is 1-4 Darcy.

17. A method according to any of the preceding claims, wherein the geological formation wherein the reservoir is present is treated to provide a mechanical strength of at least 10 MPa, in particular of 15-20 MPa.

18. A method according to any of the preceding claims, wherein at least the urea and/or calcium or other ion are applied at a rate of at least 0.075 cm/sec.

Fig. 1

Fig. 2

Fig. 3

**European Patent Office**

## EUROPEAN SEARCH REPORT

Application Number

EP 07 11 2297

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NEMATI M ET AL: "Modification of porous media permeability, using calcium carbonate produced enzymatically in situ" ENZYME AND MICROBIAL TECHNOLOGY, STONEHAM, MA, US, vol. 33, no. 5, 8 October 2003 (2003-10-08), pages 635-642, XP002372268 ISSN: 0141-0229 * the whole document * ----- | 1-18 | INV. C09K8/582 C12N11/00 |
| X | STOCKS-FISCHER S ET AL: "Microbiological precipitation of CaCO3" SOIL BIOLOGY AND BIOCHEMISTRY, PERGAMON, OXFORD, GB, vol. 31, no. 11, October 1999 (1999-10), pages 1563-1571, XP002372267 ISSN: 0038-0717 * the whole document * ----- | 1-18 | |
| X | US 5 143 155 A (FERRIS FREDERICK G [CA] ET AL) 1 September 1992 (1992-09-01) * column 2, line 40 - line 50 * * column 5, line 41 - line 55 * ----- | 1-18 | TECHNICAL FIELDS SEARCHED (IPC) C09K C12N |
| X | WO 2005/124100 A (STATOIL ASA [NO]; KOTLAR HANS KRISTIAN [NO]; HAAVIND FRODE [NO]; GOLDI) 29 December 2005 (2005-12-29) * page 20, line 22 - page 21, line 9 * ----- | 1-18 | |

The present search report has been drawn up for all claims

| Place of search | Date of completion of the search | Examiner |
|---|---|---|
| Munich | 15 November 2007 | Zimpfer, Emmanuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 07 11 2297

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2007

| Patent document cited in search report | | Publication date | Patent family member(s) | | Publication date |
|---|---|---|---|---|---|
| US 5143155 | A | 01-09-1992 | CA | 2062241 A1 | 06-09-1992 |
| WO 2005124100 | A | 29-12-2005 | AU | 2005254781 A1 | 29-12-2005 |
| | | | CA | 2569782 A1 | 29-12-2005 |
| | | | US | 2007204990 A1 | 06-09-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4506732 A
- US 4168257 A
- WO 2006066326 A
- NL 2006000598 W

**Non-patent literature cited in the description**

- **W. LOOYESTIJN.** Permeability determination through NMR detection of acoustically induced fluid oscillation. *Magnetic Resonance Imaging,* vol. 24 (9), 1187-1201